# EUROPEAN PATENT APPLICATION

(11) **EP 4 830 657 A1**
(43) Date of publication of application: **28.10.2026**
(21) Application number: 26174746.3
(22) Date of filing: 27.04.2026
(51) Int. Cl.: A61B 34/20, A61F 2/46

(54) **FEMORAL VERSION MEASUREMENT**

(30) Priority: 25.04.2025 US 202519189446
(71) Applicant: Globus Medical, Inc., Audubon, PA 19403 (US)
(72) Inventor: CAMERON, Hayden, Philadelphia, 19129 (US); OLUWASAKIN, Israel, Dresher, 19025 (US); WASHBURN II, Richard H., Wayne, 19087 (US)
(74) Representative: Morabito, Sara

(57) **Abstract**

Various aspects include a method of performing a THA procedure. In some cases, the method includes: resecting the femoral head of a patient femur; attaching a navigated trial to the resected femur; registering the attached navigated trial relative to the resected femur; and calculating the femoral version based on a position of the registered navigated trial for use in the THA procedure.

## Description

### CROSS-REFERENCE TO RELATED APPLICATIONS

The present application is a continuation of U.S. Patent Application No. 19/188,673, filed on April 24, 2025, which is incorporated herein by reference.

### TECHNICAL FIELD

This disclosure generally relates to total hip arthroplasty (THA) procedures. In particular aspects, the disclosure relates to determining a femoral version for a THA procedure.

### BACKGROUND

During a THA procedure, the femoral head is resected and replaced with a prosthetic femoral stem and head. However, conventional approaches for placing the prosthetic femoral stem and head can be inaccurate, causing complications during and/or after the THA procedure.

### SUMMARY

All examples and features mentioned below can be combined in any technically possible way.

Various implementations of the disclosure include approaches for performing a THA procedure, including calculating a femoral version.

In some particular aspects, a method of performing a THA procedure includes: resecting the femoral head of a patient femur; attaching a navigated trial to the resected femur; registering the attached navigated trial relative to the resected femur; and calculating the femoral version based on a position of the registered navigated trial for use in the THA procedure.

In some aspects, calculating the femoral version includes calculating a circumferential angle of the registered navigated trial and a posterior condylar axis of the patient femur.

In some aspects, a method further includes adjusting a position of a planned acetabular shell of an implant in response to determining that the calculated femoral version does not match a target femoral version.

In some aspects, a method further includes removing the registered navigated trial and placing a prosthetic femoral stem and head based on the calculated femoral version.

In some aspects, attaching the navigated trial includes: inserting a trial femoral stem into the patient femur; attaching a navigated instrument adapter to the inserted trial femoral stem; and attaching a navigated instrument to the navigated instrument adapter.

In some aspects, attaching the navigated instrument includes attaching the navigated instrument in a known position relative to the trial femoral stem.

In some aspects, the navigated instrument adapter includes a set of alignment features for establishing the known position of the navigated instrument relative to the trial femoral stem. In some examples, the alignment features include sets of complementary pins, slots, and/or couplers.

In some aspects, registering the navigated trial includes: using a navigation probe to palpate medial and lateral transepicondylar points (e.g., on the distal end of the femur), measuring the transepicondylar axis (TEA) of the patient femur, registering the measured TEA to at least one reference element on the navigated trial, calculating a difference between the measured TEA and a femoral neck axis associated with the navigated trial, and adding the calculated difference between the measured TEA and femoral neck axis to a known angle between the posterior condylar axis and the TEA to determine the femoral version.

In some aspects, registering the navigated trial is performed using a point cloud approach on a proximal portion of the femur.

In some aspects, the point cloud approach is performed after attaching the navigated trial to the resected femur, and includes: using a navigated instrument to establish a point cloud on the proximal portion of the femur, and registering the point cloud to a pre-operative computed tomography (CT) image-based model of the patient. In some examples, because the registration is performed using the navigated trial, the location of the prosthesis femoral neck axis is known, and the prosthesis femoral version can be calculated using the posterior condylar axis, e.g., as identified in the CT image-based model.

In some aspects, registering the navigated trial is performed using an intra-operative fluoroscopy approach.

In some aspects, the intra-operative fluoroscopy approach is performed after attaching the navigated trial to the resected femur, and includes: obtaining intra-operative fluoroscopy images of a distal portion of the patient femur, and registering the distal portion of the femur to a pre-operative computed tomography (CT) image-based model of the patient. In some examples, because the registration is performed using the navigated trial, the location of the prosthesis femoral neck axis is known, and the prosthesis femoral version can be calculated using the posterior condylar axis, e.g., as identified in the CT image-based model.

In further particular aspects, a version measurement device (VMD) is configured for attachment to a resected femoral head region of a patient femur. In certain cases, the VMD is referred to as a "navigated trial." In particular aspects, the VMD (or navigated trial) includes: a trial femoral stem; a navigated instrument; and a navigated instrument adapter for coupling the navigated instrument to the trial femoral stem.

In some aspects, the navigated instrument further includes a set of reference elements.

In some aspects, the set of reference elements are optically detectable.

In some aspects, the navigated instrument adapter includes a set of alignment features for establishing a known position of the navigated instrument relative to the trial femoral stem.

In some aspects, the set of alignment features include male/female couplers, e.g., pins, slots, and/or tabs.

In some aspects, the known position of the navigated instrument relative to the trial femoral stem aids in calculating a femoral version of the patient femur during a total hip arthroplasty (THA) procedure.

In some aspects, the trial femoral stem is configured to be placed in a resected femoral head portion of the patient femur.

Particular aspects include a process flow that can include: i) resecting the femoral head of a patient during a THA procedure; ii) attaching a navigated trial (also called a version measurement device (VMD)) to the resected femoral head region; iii) registering the navigated trial relative to the patient femur; iv) calculating a femoral version; v) comparing the calculated femoral version with a target femoral version; vi) adjusting at least one alignment parameter if the calculated femoral version deviates from the target; and vii) replacing the navigated trial (VMD) with a femoral implant if the calculated femoral version aligns with the target.

Two or more features described in this disclosure, including those described in this summary section, may be combined to form implementations not specifically described herein.

The details of one or more implementations are set forth in the accompanying drawings and the description below. Other features, objects and advantages will be apparent from the description and drawings, and from the claims.

### DESCRIPTION OF THE DRAWINGS

FIG. 1 is a schematic depiction illustrating calculation of a native femoral version according to various implementations.
FIG. 2 is a schematic depiction illustrating calculation of a femoral version of an implant according to various implementations.
FIG. 3 is a schematic depiction of a femur and a portion of a navigated trial according to various implementations.
FIG. 4 is a schematic depiction of portions of a navigated trial according to various implementations.
FIG. 5 is a schematic depiction of processes in coupling a navigated trial to a femur according to various implementations.
FIG. 6 illustrates a process in registering a navigated trial according to various implementations.
FIG. 7 illustrates measurement approaches for femoral implants according to various implementations.
FIG. 8 illustrates a process in registering a navigated trial according to various additional implementations.
FIG. 9 illustrates a process in registering a navigated trial according to various additional implementations.
FIG. 10 is a flow diagram illustrating processes in a method according to various implementations.

It is noted that the drawings of the various implementations are not necessarily to scale. The drawings are intended to depict only typical aspects of the disclosure, and therefore should not be considered as limiting the scope of the invention. In the drawings, like numbering represents like elements between the drawings.

### DETAILED DESCRIPTION

As noted herein, various aspects of the disclosure generally relate to total hip arthroplasty (THA) procedures and related constructs and devices used in such procedures. In particular cases, the disclosure includes approaches for performing a THA procedure, including calculating a femoral version. In further cases, the disclosure includes a navigated trial (also called a version measurement device, or VMD) for measuring a femoral version during a THA procedure.

Commonly labeled components in the FIGURES are considered to be substantially equivalent components for the purposes of illustration, and redundant discussion of those components is omitted for clarity.

As noted herein, aspects of the disclosure relate to THA procedures. The THA procedure is known in the art, and is not discussed in detail herein. Additional details of THA procedures are described, for example, in Patent App. No. 15/180,126, filed June 13, 2016 (U.S. 10,842,453), Patent App. No. 15/157,444, filed May 18, 2016 (U.S. Pub. No. 2016/0256225), US Patent Application No. 18/430,077 (Docket No. ROBOT.134.0002), entitled "Computer Assisted Pelvic Surgery Navigation", filed on February 1, 2024), US Patent Application No. 18/743,388 (Docket No. GM23-276-EFlex-THA/IDR-23-131), entitled "PATIENT REGISTRATION FOR TOTAL HIP ARTHROPLASTY PROCEDURE USING PRE-OPERATIVE COMPUTED TOMOGRAPHY (CT) AND INTRA-OPERATIVE FLUOROSCOPY", filed on June 14, 2024), and US Patent Application No. 18/743,615 (Docket No. GM23-276-EFlex-THA/IDR-23-151), entitled "PATIENT REGISTRATION FOR TOTAL HIP ARTHROPLASTY PROCEDURE USING PRE-OPERATIVE COMPUTED TOMOGRAPHY (CT) AND POINT CLOUD DATA", filed on June 14, 2024), each of which is incorporated herein by reference.

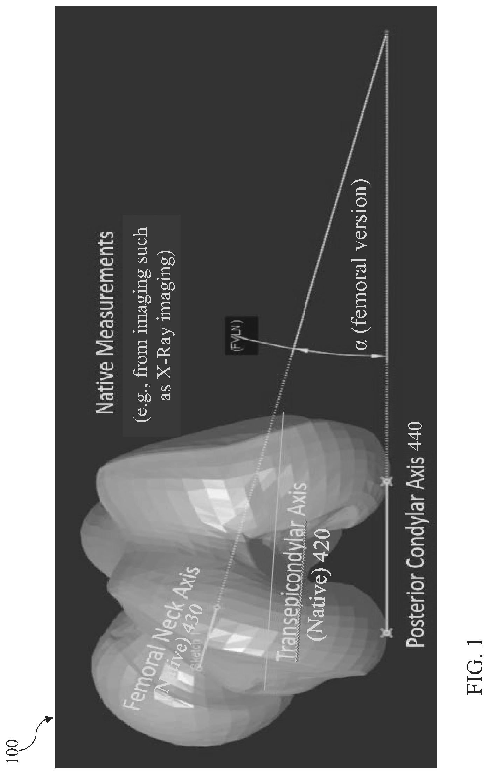

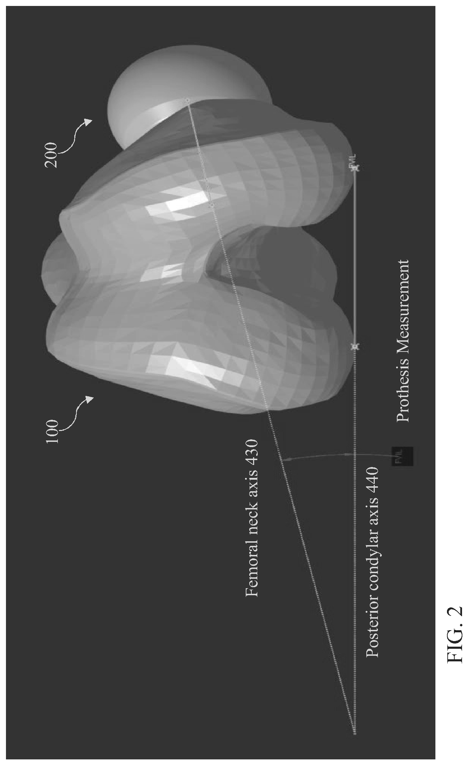

In contrast to conventional approaches and devices, various implementations include devices (e.g., navigated trials, also called version measurement devices (VMDs)) and related approaches for determining a femoral version during a THA procedure. As illustrated in FIG. 1, the native femoral version is defined as the angle (α) between the femoral neck and the posterior condylar axis of the femur 100. During a THA procedure, the femoral head is resected and replaced with a prosthesic femoral stem and head. Various aspects of the disclosure provide for measurement of the femoral version for aiding in placement of the prosthesis. According to the disclosed implementations, the femoral version is defined as the angle between the femoral neck of the prosthesis and the posterior condylar axis of the femur. Prosthesis measurements are illustrated, for example, on the prosthesis 200 in FIG. 2.

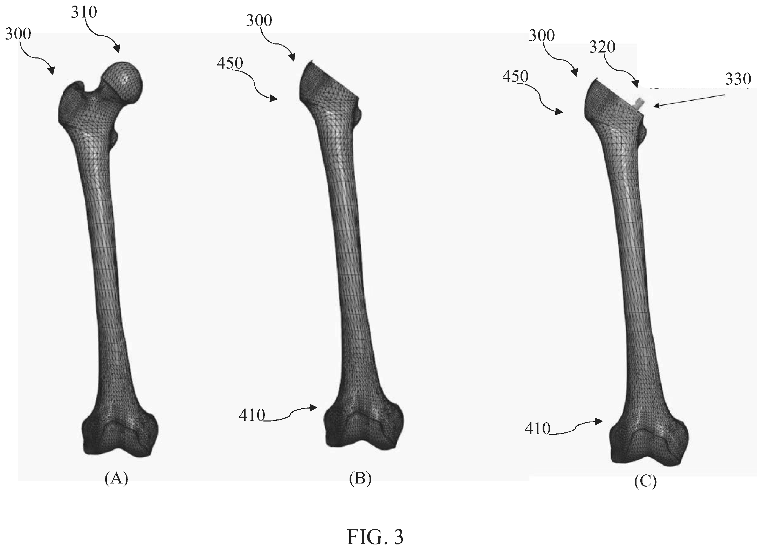

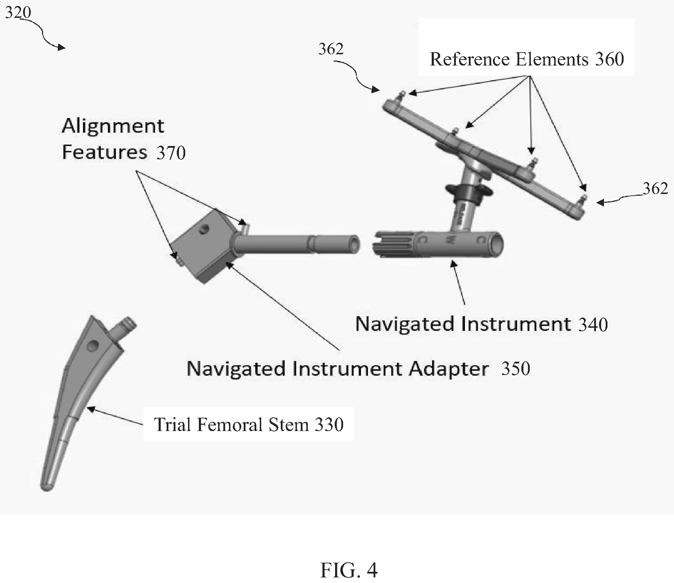

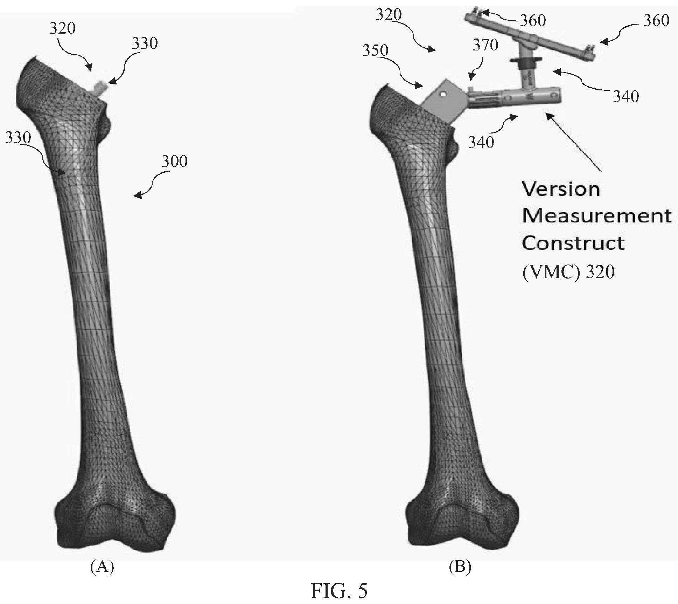

FIG. 3 illustrates processes in a method according to various implementations. (A) shows a femur 300 prior to resection, (B) shows the femur 300 with the femoral head 310 resected, and (C) shows a portion of a navigated trial 320 attached to the resected femur 300. In particular cases, the portion 330 of the navigated trial is referred to as a trial femoral stem 330, and is one of several components in the navigated trial 320, e.g., as illustrated schematically in FIG. 4. In certain cases, the navigated trial 320 is referred to as a version measurement device (VMD), and can include the trial femoral stem 330, a navigated instrument 340, and a navigated instrument adapter 350 for coupling the navigated instrument 340 to the trial femoral stem 330. FIG. 5 illustrates the trial femoral stem 330 (A) coupled with the resected femur 300 and the remainder of the navigated trial 320 coupled with the trial femoral stem 330 (B). In some cases, the trial femoral stem 330 is attached first (e.g., inserted into a cavity formed in the resected femur 300), followed by the navigated instrument adapter 350 (coupled with the stem 330), and then the navigated instrument 340 (coupled with the adapter 350). In other cases, the navigated instrument 340 and adapter 350 are coupled prior to joining with the trial femoral stem 330.

In particular cases, as shown in FIGS. 4 and 5, the navigated instrument 340 further includes a set of reference elements 360, e.g., optically detectable reference elements. In particular cases, reference elements 360 are connected via mounts 362 on arms of the navigated instrument 340, e.g., in an array. In some cases, reference elements 360 are referred to as tracking markers. Use of reference elements 360 to track the location of a device are described, for example, in US Patent Application No. 18/743,388 (Docket No. GM23-276-EFlex-THA/IDR-23-131), and US Patent Application No. 18/743,615 (Docket No. GM23-276-EFlex-THA/IDR-23-151), previously incorporated by reference herein. In some aspects, the navigated instrument adapter 350 includes a set of alignment features 370 for establishing a known position of the navigated instrument 340 relative to the trial femoral stem 330. In some aspects, the set of alignment features 370 include male/female couplers, e.g., pins, slots, and/or tabs. In certain aspects, the known position of the navigated instrument 340 relative to the trial femoral stem 330 aids in calculating a femoral version of the patient femur during the total hip arthroplasty (THA) procedure. That is, the navigated instrument 340 can be attached in a known position relative to the trial femoral stem 330.

In particular implementations, after attaching the navigated trial 320 to the resected femur 300, the inserted (attached) navigated trial 320 is registered to the resected femur 300.

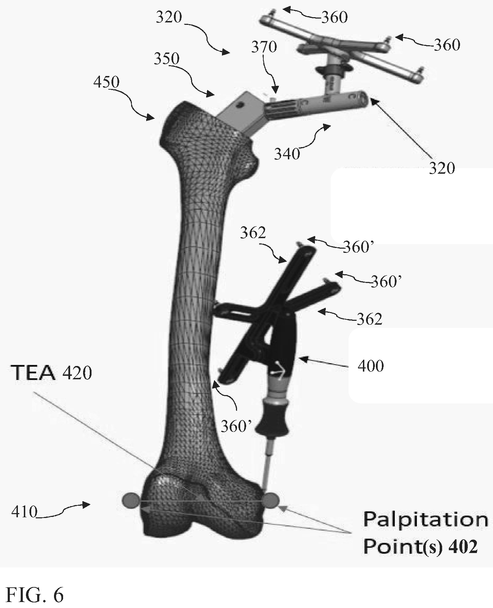

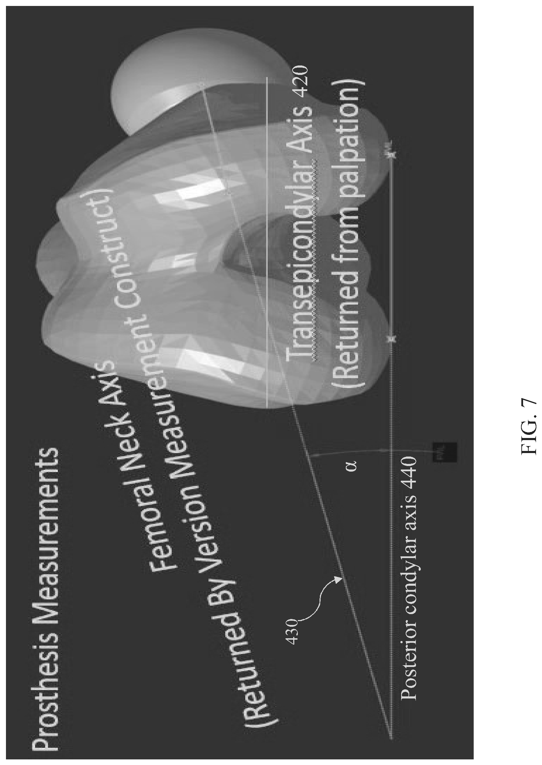

In some cases, registration can be performed using a navigation probe 400, e.g., as illustrated schematically in FIG. 6. Navigation probe 400 can include a palpitation device configured to trace or contact (also called "surface painting") a plurality of surfaces on an anatomical feature, e.g., points on a femur 300. Palpitation based data gathering, e.g., with a navigation probe 400, is further described in US Patent Application No. 18/743,388 (Docket No. GM23-276-EFlex-THA/IDR-23-131), and US Patent Application No. 18/743,615 (Docket No. GM23-276-EFlex-THA/IDR-23-151), previously incorporated by reference herein. In these cases, registration is performed with navigation probe 400 to palpitate medial and lateral transepicondylar points 402 (e.g., on the distal end 410 of the femur 300), measuring the transepicondylar axis (TEA) 420 of the patient femur 300. The measured TEA 420 is then registered (e.g., using reference elements 360' on navigation probe 400) to at least one reference element 360 on the navigated trial 320 (e.g., one or more reference elements 360). Following registering the TEA 420 to the reference element(s) 360 on the navigated trial 320, the process includes calculating a difference between the measured TEA 420 and a femoral neck axis 430 associated with the navigated trial 320 (FIG. 7). That calculated difference between the measured TEA 420 and femoral neck axis 430 is then added to a known angle between the posterior condylar axis 440 (FIG. 1) and the TEA 420 to determine the femoral version.

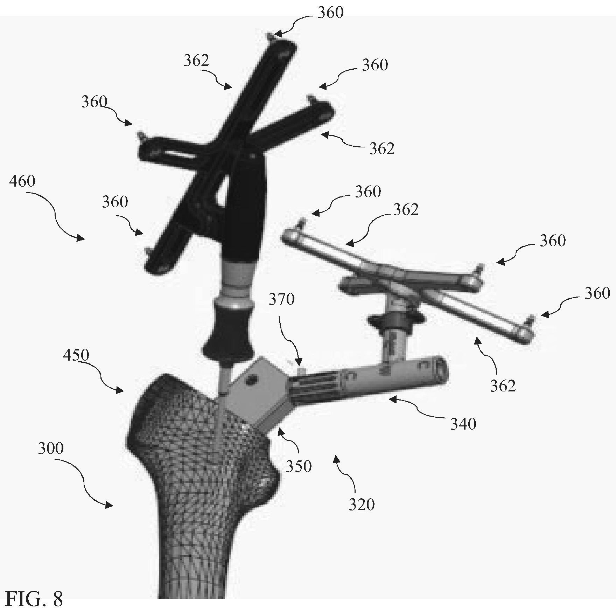

In additional cases, registration is performed using a point cloud approach on a proximal portion 450 of the femur 300 (shown schematically in FIG. 8). In some aspects, the point cloud approach is performed after attaching the navigated trial 320 to the resected femur 300, and includes: a) using a navigated instrument 460 to establish a point cloud on the proximal portion 450 of the femur 300, and b) registering the point cloud to a pre-operative computed tomography (CT) image-based model of the patient. Point cloud data gathering, e.g., with a navigated instrument 450, is further described in US Patent Application No. 18/743,388 (Docket No. GM23-276-EFlex-THA/IDR-23-131), and US Patent Application No. 18/743,615 (Docket No. GM23-276-EFlex-THA/IDR-23-151), previously incorporated by reference herein. In some examples, because the registration is performed using the navigated trial 320, the location of the prosthesis femoral neck axis 430 (FIG. 7) is known, and the prosthesis femoral version can be calculated using the posterior condylar axis 440, e.g., as identified in the CT image-based model.

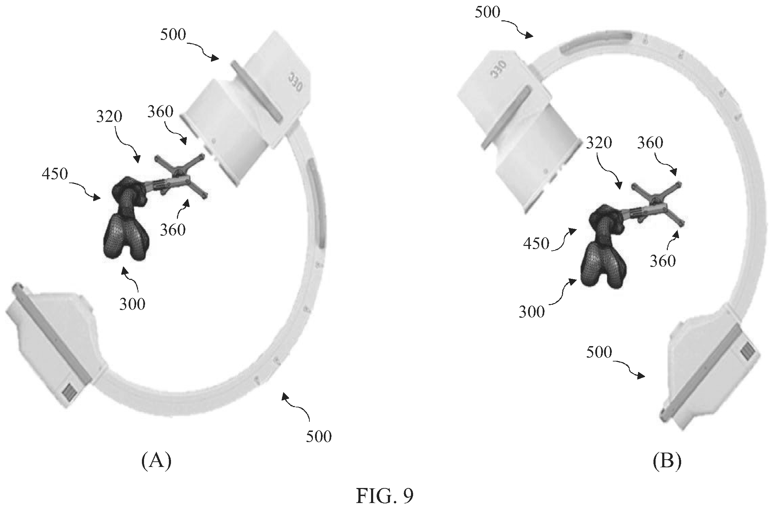

In further examples, registration is performed using an intra-operative fluoroscopy approach, as illustrated in the schematic depiction of FIG. 9, e.g., in a first position (A) and second position (B), among others. In certain of these aspects, the intra-operative fluoroscopy approach is performed after attaching the navigated trial 320 to the resected femur 300, and includes obtaining intra-operative fluoroscopy images (e.g., using fluoroscopy imaging system 500 in one or more positions (A), (B), etc.) of a distal portion 410 of the patient femur 300. The distal portion 410 of the femur 300 can be registered to a pre-operative computed tomography (CT) image-based model of the patient. In some examples, because the registration is performed using the navigated trial 320, the location of the prosthesis femoral neck axis 430 (FIG. 7) is known, and the prosthesis femoral version can be calculated using the posterior condylar axis, e.g., as identified in the CT image-based model.

Further implementations include, after registering the navigated trial 320, calculating the femoral version based on the position of the registered navigated trial, e.g., for use in the THA procedure. In some aspects, calculating the femoral version includes calculating a circumferential angle of the registered navigated trial 320 and a posterior condylar axis 440 of the patient femur.

In particular cases, a process further includes adjusting a position of a planned acetabular shell of an implant in response to determining that the calculated femoral version (angle α, FIG. 7) does not match a target femoral version, e.g., the target (or native) femoral version (angle α, FIG. 1). In particular cases, the calculated femoral version for the implant is iteratively compared with the target femoral version (e.g., an optimal femoral version angle) until the calculated femoral version for the implant aligns with the target femoral version (e.g., within an acceptable margin such as 1-2 percent). In some aspects, the method further includes removing the registered navigated trial 320 and placing a prosthetic femoral stem and head based on the calculated femoral version, e.g., when the calculated femoral version of the implant aligns with the target femoral version.

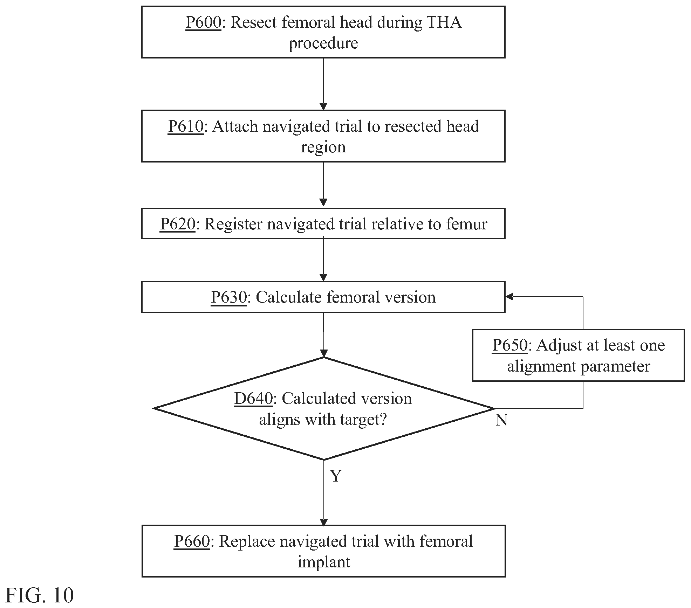

FIG. 10 is a flow diagram illustrating processes in performing a THA procedure according to various implementations. FIG. 10 is referred to in concert with FIGS. 1-9 herein. As shown, processes can include:
P600: resecting the femoral head of a patient during a THA procedure;
P610: attaching a navigated trial (also called a version measurement device (VMD)) to the resected femoral head region;
P620: registering the navigated trial relative to the patient femur;
P630: calculating a femoral version;
D640: determining whether the calculated femoral version aligns with a target femoral version;
P650: adjusting at least one alignment parameter, in response to determining that the calculated femoral version fails to align with target femoral version (No to D640). In some cases, the surgeon or other operator may decide to adjust at least one alignment parameter to compensate for the femoral version. One non-limiting example parameter that the surgeon may adjust is the angle of the acetabular shell to be inserted. If, for example, the femoral version is three degrees offset from what the surgeon considers acceptable (e.g., the target femoral version), then the angle of the acetabular shell may be adjusted by the same three degrees to compensate; and
P660: replacing the navigated trial (VMD) with a femoral implant if the calculated femoral version aligns with the target femoral version (Yes to D640).

As noted herein, particular implementations can improve the efficacy of THA procedures, e.g., by enhancing accuracy of placement of implants.

### Further Definitions and Embodiments:

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

Other embodiments not specifically described herein are also within the scope of the following claims. Elements of different implementations described herein may be combined to form other embodiments not specifically set forth above. Elements may be left out of the structures described herein without adversely affecting their operation. Furthermore, various separate elements may be combined into one or more individual elements to perform the functions described herein.

The invention could be defined interalia by the following examples
1. A version measurement device (VMD) configured for attachment to a resected femoral head region of a patient femur, the VMD comprising:
   a trial femoral stem;
   a navigated instrument; and
   a navigated instrument adapter for coupling the navigated instrument to the trial femoral stem.
2. The VMD of Example 1, wherein the navigated instrument further includes a set of reference elements.
3. The VMD of Example 2, wherein the set of reference elements are optically detectable.
4. The VMD of Example 1, wherein the navigated instrument adapter includes a set of alignment features for establishing a known position of the navigated instrument relative to the trial femoral stem.
5. The VMD of Example 4, wherein the set of alignment features include male/female couplers.
6. The VMD of Example 4, wherein the known position of the navigated instrument relative to the trial femoral stem aids in calculating a femoral version of the patient femur during a total hip arthroplasty (THA) procedure.
7. The VMD of Example 1, wherein the trial femoral stem is configured to be placed in a resected femoral head portion of the patient femur.
8. A femoral version measurement system comprising:
   a navigated probe including an optical array trackable by a tracking device and a tip for palpating a surface of a bone while the optical array is tracked by the tracking device; and
   a version measure device (VMD) including:
      a trial femoral stem configured for attachment to a resected femoral head of a patient femur,
      a navigated instrument having a plurality of optical markers for tracking by the tracking device; and
      a navigated instrument adapter for coupling the navigated instrument to the trial femoral stem at a predetermined pose.
9. The system of Example 8, wherein the navigated instrument adapter includes a set of alignment features for establishing the predetermined pose of the navigated instrument relative to the trial femoral stem.
10. The system of Example 9, wherein the set of alignment features include a female coupler that couples to a male coupler on the trial femoral stem at a known angular position.
11. The system of Example 8, wherein the trial femoral stem is configured to be inserted into a resected femoral head of the patient femur.

## Claims

1. A version measurement device (VMD) configured for attachment to a resected femoral head region of a patient femur, the VMD comprising:
- a trial femoral stem;
- a navigated instrument; and
- a navigated instrument adapter for coupling the navigated instrument to the trial femoral stem.

2. The VMD of claim 1, wherein the navigated instrument further includes a set of reference elements.

3. The VMD of claim 2, wherein the set of reference elements are optically detectable.

4. The VMD of any one of the preceding claims, wherein the navigated instrument adapter includes a set of alignment features for establishing a known position of the navigated instrument relative to the trial femoral stem.

5. The VMD of claim 4, wherein the set of alignment features include male/female couplers.

6. The VMD of claim 4 or 5, wherein the known position of the navigated instrument relative to the trial femoral stem aids in calculating a femoral version of the patient femur during a total hip arthroplasty (THA) procedure.

7. The VMD of any one of the preceding claims, wherein the trial femoral stem is configured to be placed in a resected femoral head portion of the patient femur.

8. A femoral version measurement system comprising:
- a tracking device for tracking a probe
- a navigated probe including an optical array trackable by the tracking device and a tip configured for palpating a surface of a bone while the optical array is tracked by the tracking device; and
- a version measure device (VMD) according to any one of the preceding claims wherein the
navigated instrument has a plurality of optical markers for tracking by the tracking device; and
- the navigated instrument adapter is configured for coupling the navigated instrument to the trial femoral stem at a predetermined pose.

9. The system of claim 8, wherein the navigated instrument adapter includes a set of alignment features for establishing the predetermined pose of the navigated instrument relative to the trial femoral stem.

10. The system of claim 9, wherein the set of alignment features include a female coupler that couples to a male coupler on the trial femoral stem at a known angular position.

11. The system of any one of claims 8-10, wherein the trial femoral stem is configured to be inserted into a resected femoral head of the patient femur.
